(19) Europäisches Patentamt
European Patent Office
Office européen des brevets

(11) EP 0 273 013 B2

(12) NEUE EUROPÄISCHE PATENTSCHRIFT

(45) Veröffentlichungstag und Bekanntmachung des Hinweises auf die Entscheidung über den Einspruch:
**24.07.1996 Patentblatt 1996/30**

(45) Hinweis auf die Patenterteilung:
**07.04.1993 Patentblatt 1993/14**

(21) Anmeldenummer: **87810767.1**

(22) Anmeldetag: **18.12.1987**

(51) Int Cl.[6]: **C08K 5/37**, C07C 321/00, C08L 21/00, C10M 135/22

# (54) Substituierte Phenole als Stabilisatoren

Substituted phenols as stabilizers

Phénols substitués utilisables comme des stabilisateurs

(84) Benannte Vertragsstaaten:
**BE DE FR GB IT NL**

(30) Priorität: **24.12.1986 CH 5235/86**

(43) Veröffentlichungstag der Anmeldung:
**29.06.1988 Patentblatt 1988/26**

(73) Patentinhaber: **CIBA-GEIGY AG**
**4002 Basel (CH)**

(72) Erfinder:
- **Meier, Hans Rudolf, Dr.**
  **CH-1723 Marly (CH)**
- **Evans, Samuel, Dr.**
  **CH-1723 Marly (CH)**
- **Dubs, Paul, Dr.**
  **CH-1723 Marly (CH)**

(56) Entgegenhaltungen:
**US-A- 2 415 833** **US-A- 3 553 270**
**US-A- 4 707 300**

- **CHEMICAL ABSTRACTS, Band 89, Nr. 4, 24. Juli 1978, Seite 161, Zusammenfassung Nr. 27167j, Columbus, Ohio, US; F.A. ABDULLAEVA et al.: "Phenol derivatives as oil stabilizers", & NEFTEKHIMIYA 1978, 18 (1), 124-32**
- **CHEMICAL ABSTRACTS, Band 83, Nr. 10, 8. September 1975, Seite 20, Zusammenfassung Nr. 79881h; Columbus, Ohio, US; F.A. ABDULLAEVA et al.: "Synthesis of screened phenols", & AZERB. KHIM. ZH. 1973, (5-6), 66-9**
- **CHEMICAL ABSTRACTS, Band 86, Nr. 1, 3. Januar 1977, Seite 437, Zusammenfassung Nr. 5067n, Columbus, Ohio, US; F.A. ABDULLAEVA et al.: "Synthesis of new organosulfur derivatives of shielded phenols", & TEZISY DOKL. NAUCHN. SESS. KHIM. TEKHNOL. ORG. SOEDIN. SERY SERNISTYKH NEFTEI, 13th 1974, 140-1**
- **"Elastomers" definition - The Condesed Chem. Dictionary, 9.ed.,G.Hawley, Van Nordstrand, 1977, p.335**
- **"Elastomers" definition - Encyclopedia of Chemistry, 3rd Ed., C.A.Hampel, G.G.Hawley, Van Nordstrand, 1966**

Bemerkungen:
Die Akte enthält technische Angaben, die nach dem Eingang der Anmeldung eingereicht wurden und die nicht in dieser Patentschrift enthalten sind.

EP 0 273 013 B2

## Beschreibung

Die vorliegende Erfindung betrifft Zusammensetzungen enthaltend substituierte Bis-(merkaptomethyl)-phenole als Stabilisatoren sowie neue Bis-(merkaptomethyl)-phenole.

Merkaptomethylhaltige Phenole sind bekannt als Stabilisatoren. So sind beispielsweise in der US-PS 2 417 118 einige Bis-(merkaptomethyl)-phenole aufgezählt, jedoch ohne genaue Angaben über die substituierten Stellen am Phenol. Diese Phenole eignen sich als Additive für Schmierstoffe, insbesondere in Motorenölen.

Aus der US-PS 3 227 667 sind 2,6-Bis-(alkoxycarbonylalkylenthiomethyl)-4-alkylphenole als Antioxidantien für Polyolefine bekannt.

Nach der EP-A-0 165 209 sind beispielsweise 2,4-Bis-(merkaptomethyl)-6-alkylphenole als Stabilisatoren für organische Polymere und Schmierstoffe geeignet.

Es sind ferner einige spezifische 2,6-Bis-(merkaptomethyl)-phenole als Zwischenprodukte bekannt, wie zum Beispiel 2,6-Bis-(ethylthiomethyl)-4-methyl-phenol (DE-A1-2 838 273), 2,6-Bis-(butylthiomethyl)-4-methyl-phenol [I.W. Rudermann, E.M. Fettes, J. Am. Chem. Soc. 71 (1949), 2264] sowie 2,6-Bis-(phenylthiomethyl)- und 2,6-Bis-(benzylthiomethyl)-4-methyl-phenol [Abdullaeva, C.A. Vol. 83 (1975), 79881 h].

Im US Patent 4,215,833 wird die Herstellung von 2,6-Bis(amylthiomethyl)-4-octyl-phenol und dessen Verwendung als Züsatz in Schmiermitteln beschrieben.

Es besteht weiterhin ein Bedarf an wirksamen Stabilisatoren für Materialien, welche gegen thermischen, oxidativen oder lichtinduzierten Abbau empfindlich sind.

Gegenstand der Erfindung ist daher eine Zusammensetzung enthaltend ein Elastomer oder einen Schmierstoff und mindestens eine Verbindung der Formeln I oder/und II

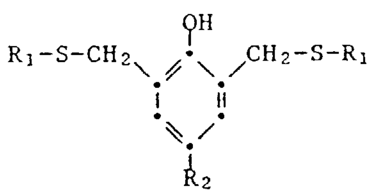 (I) 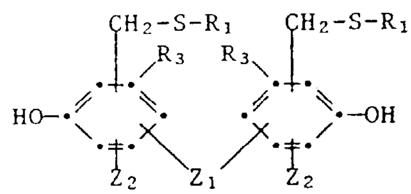 (II),

worin

die Reste $R_1$ gleich oder verschieden sind und unabhängig voneinander $C_8$-$C_{20}$-Alkyl bedeuten, welches unsubstituiert ist oder durch 1 oder 2 Hydroxylgruppen substituiert oder/und durch -O- unterbrochen ist, oder $C_1$-$C_4$-Alkylen-COO$R_2'$, $C_1$-$C_4$-Alkylen-CO-N$R_2''R_4$, $C_5$-$C_{12}$-Cycloalkyl oder Phenyl-$C_1$-$C_4$-alkyl bedeuten,

$R_2$, $R_2'$ und $R_2''$ unabhängig voneinander $C_1$-$C_{20}$-Alkyl, Allyl, Methallyl, Propargyl, $C_5$-$C_{12}$-Cycloalkyl, Phenyl oder Benzyl sind,

$R_3$ Wasserstoff, $C_1$-$C_{20}$-Alkyl, $C_2$-$C_{18}$-Alkenyl oder Halogen bedeutet,

$R_4$ Wasserstoff, $C_1$-$C_{20}$-Alkyl oder $C_2$-$C_{18}$-Alkenyl ist,

$Z_1$ für -S- oder -C($Z_3$)($Z_4$)- steht,

$Z_2$ Wasserstoff, $C_1$-$C_{20}$-Alkyl oder -$CH_2$-S-$R_1$ ist,

$Z_3$ und $Z_4$ unabhängig voneinander Wasserstoff oder Methyl bedeuten,

mit der Massgabe, dass die Phenole der Formel II in m-Stellung zur OH-Gruppe frei von der funktionellen Gruppe -$CH_2$-S-$R_1$ sind.

$R_1$, $R_2$, $R_2'$, $R_2''$, $R_3$, $R_4$ und $Z_2$ bedeuten als $C_1$-$C_{20}$- bzw. $C_8$-$C_{20}$-Alkyl beispielsweise Methyl, Ethyl, n-Propyl, Isopropyl, n-Butyl, sek-Butyl, t-Butyl, n-Pentyl, Isopentyl, n-Hexyl, 1,1-Dimethylbutyl, n-Heptyl, n-Octyl, 2-Ethylhexyl, n-Nonyl, n-Decyl, 1,1,3,3-Tetramethylbutyl, 1,1,3,3-Tetramethylhexyl, n-Undecyl, n-Dodecyl, 1,1,3,3,5,5-Hexamethylhexyl, 2,2,4,6,6-Pentamethylhept-4-yl, n-Tetradecyl, n-Hexadecyl, n-Octadecyl oder n-Eicosyl, je nach Anzahl der C-Atome.

$R_1$ ist vorzugsweise ein geradkettiges $C_8$-$C_{20}$-Alkyl, insbesondere $C_8$-$C_{12}$-Alkyl, wie z.B. n-Octyl, n-Decyl oder n-Dodecyl.

$R_2$, $R_2'$ und $R_2''$ sind vorzugsweise Methyl, Ethyl oder ein verzweigtes $C_3$-$C_{20}$-Alkyl, wie z.B. Isopropyl, t-Butyl, t-Amyl, 2-Ethylhexyl, 1,1,3,3-Tetramethylbutyl, 1,1,3,3-Tetramethylhexyl oder 1,1,3,3,5,5-Hexamethylhexyl und insbesondere Methyl, t-Butyl, 2-Ethylhexyl und 1,1,3,3-Tetramethylbutyl.

$R_1$ als durch ein oder zwei Hydroxylgruppen substituiertes $C_8$-$C_{20}$-Alkyl bedeutet beispielsweise 2-Hydroxyoctyl, 2-Hydroxydecyl, 2-Hydroxydodecyl, 2-Hydroxytetradecyl, 2-Hydroxyhexadecyl, 2-Hydroxyoctadecyl, 2-Hydroxyeicosyl oder 2,3-Dihydroxypropyl.

$R_1$ als durch -O- unterbrochenes Alkyl kann eine oder mehrere, z.B. 1 bis 6, insbesondere 1 oder 2 -O-Unterbrechungen aufweisen und bedeutet beispielsweise 3,6,9-Trioxadecyl oder 3,6,9,12,15,18-Hexaoxanonadecyl.

$R_3$ und $R_4$ als $C_2$-$C_{18}$-Alkenyl bedeuten beispielsweise Vinyl, Allyl, But-3-enyl, Pent-4-enyl, Hex-5-enyl, Oct-7-enyl, Dec-9-enyl, Dodec-11-enyl oder Octadec-17-enyl. Bevorzugt werden Vinyl oder Allyl.

$R_1$, $R_2$, $R_2'$ und $R_2''$ als $C_5$-$C_{12}$-Cycloalkyl bedeuten beispielsweise Cyclopentyl, Cyclohexyl, Cycloheptyl, Cyclooctyl, Cyclononyl, Cyclodecyl, Cycloundecyl oder Cyclododecyl. Bevorzugt werden $C_5$-$C_7$-Cycloalkyl, und $R_1$ ganz besonders als Cyclohexyl.

$R_1$ als Phenyl-$C_1$-$C_4$-alkyl bedeutet beispielsweise Benzyl, Phenylethyl, $\alpha$-Methylbenzyl oder $\alpha,\alpha$-Dimethylbenzyl. Bevorzugt wird Benzyl.

$R_3$ als Halogen bedeutet beispielsweise Chlor oder Brom, insbesondere Chlor.

Bevorzugt werden Zusammensetzungen, welche ein Elastomer und mindestens eine Verbindung der Formeln I oder/und II enthalten.

Ebenfalls bevorzugt werden Zusammensetzungen enthaltend einen Schmierstoff und mindestens eine Verbindung der Formeln I oder/und II, worin $R_2$ Methyl, Ethyl oder ein verzweigtes $C_3$-$C_{20}$-Alkyl, Allyl, Methallyl, Propargyl oder Benzyl ist und $R_1$, $R_3$, $Z_1$ und $Z_2$ die zuvor genannte Bedeutung haben.

Von besonderem Interesse sind Zusammensetzungen, welche ein Elastomer oder einen Schmierstoff und mindestens eine Verbindung der Formel I oder/und II enthalten, worin die Reste $R_1$ gleich oder verschieden sind und unabhängig voneinander $C_8$-$C_{20}$-Alkyl oder $C_1$-$C_4$-Alkylen-$COOR_2'$ bedeuten, $R_2$ und $R_2'$ für $C_1$-$C_{20}$-Alkyl stehen, $Z_1$ $-CH_2-$ oder $-C(CH_3)_2-$ ist und $Z_2$ die zuvor genannte Bedeutung hat.

Besonders bevorzugt werden Zusammensetzungen, welche ein Elastomer oder einen Schmierstoff und mindestens eine Verbindung der Formel I enthalten, worin die Reste $R_1$ unabhängig voneinander für $C_8$-$C_{20}$-Alkylgruppen stehen, welche unsubstituiert sind, oder durch 1 oder 2 Hydroxylgruppen substituierte $C_1$-$C_{20}$-Alkylgruppen bedeuten, und ganz besonders bevorzugt werden solche Zusammensetzungen, worin die Reste $R_1$ die gleiche Bedeutung haben und insbesondere für $C_8$-$C_{12}$-Alkylgruppen stehen.

Von besonderer Bedeutung sind Zusammensetzungen, welche ein Elastomer oder einen Schmierstoff und mindestens eine Verbindung der Formel IIa

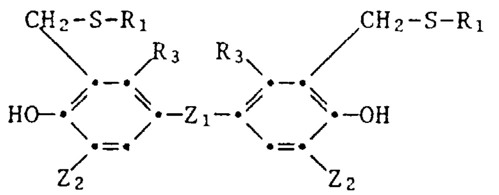

(IIa)

enthalten, worin $R_1$, $R_3$, $Z_1$ und $Z_2$ die zuvor genannte Bedeutung haben, und insbesondere solche Zusammensetzungen, worin $R_3$ Wasserstoff und $Z_1$ $-C(Z_3)(Z_4)-$ bedeuten, wobei $Z_3$ und $Z_4$ die zuvor genannte Bedeutung haben.

Ganz besonders bevorzugt sind Zusammensetzungen enthaltend eine Verbindung der Formel IIa, worin $Z_2$ den Rest $-CH_2-S-R_1$ bedeutet.

Als Beispiele für Vertreter von Verbindungen der Formeln I und II, welche in den erfindungsgemässen Zusammensetzungen enthalten sein können, sind die nachfolgend aufgezählten Substanzen anzusehen:

2,6-Bis(2'-methylaminocarbonylethylthiomethyl)-4-phenyl-phenol,
2,6-Bis(N,N-diethylaminocarbonyl-ethylthiomethyl)-4-allyl-phenol,
2,6-Bis(n-octylthiomethyl)-4-methyl-phenol,
2,6-Bis(t-octylthiomethyl)-4-t-butyl-phenol,
2,6-Bis(t-dodecylthiomethyl-4-t-octyl-phenol,
2,6-Bis(benzylthiomethyl)-6-methyl-phenol,
2,6-Bis(2'-ethylhexyloxycarbonylmethyl-thiomethyl)-4-cyclohexylphenol,
2,6-Bis(n-octadecyloxycarbonylmethyl-thiomethyl)-4-propargyl-phenol,
2,6-Bis[2'-(2"-ethylhexyloxycarbonyl)-ethylthiomethyl]-4-t-butyl-phenol,
2,2-Bis-[4',4"-dihydroxy-3',3",5',5"-tetrakis(octylthiomethyl)-phenol]-propan,
2,2-Bis-[4',4"-dihydroxy-3',3",5',5"-tetrakis-(dodecylthiomethyl)-phenyl]-methan,
Bis-[4',4"-dihydroxy-3',3",5',5"-tetrakis(2-ethylhexyloxycarbonylmethylthiomethyl)-phenyl]-thioether.

Als Elastomeres können die erfindungsgemässen Zusammensetzungen beispielsweise folgende Materialien enthalten:

1. Polydiene, wie beispielsweise Polybutadien, Polyisopren oder Polychloropren; Blockpolymere, wie beispiels-

[1] t-Octyl ist 1,1,3,3-Tetramethylbutyl
[2] t-Dodecyl ist ein Gemisch aus 1,1,3,3,5,5-Hexamethylhexyl und 1,1,4,6,6-Pentamethylhept-(4)-yl

weise Styrol/Butadien/Styrol, Styrol/Isopren/Styrol oder Acrylnitril/Butadien-Copolymere.

2. Copolymere von Mono- und Diolefinen untereinander oder mit anderen Vinylmonomeren, wie z.B. Aethylen-Alkylacrylat-Copolymere, Aethylen-Alkylmethacrylat-Copolymere, Aethylen-Vinylacetat-Copolymere sowie Terpolymere von Aethylen mit Propylen und einem Dien, wie Hexadien, Dicyclopentadien oder Aethylidennorbornen.

3. Halogenhaltige Polymere, wie z.B. Polychloropren, Chlorkautschuk, chloriertes oder chlorsulfoniertes Polyäthylen, Epichlorhydrin-Homo- und -Copolymere, Chlortrifluoroäthylen-Copolymere, Polymere aus halogenhaltigen Vinylverbindungen, wie z.B. Polyvinylidenchlorid, Polyvinylidenfluorid; sowie deren Copolymere, wie Vinylchlorid-Vinylidenchlorid, Vinylchlorid-Vinylacetat oder Vinylidenchlorid-Vinylacetat.

4. Polyurethane, die sich von Polyäthern, Polyestern und Polybutadien mit endständigen Hydroxylgruppen einerseits und aliphatischen oder aromatischen Polyisocyanaten andererseits ableiten sowie deren Vorprodukte.

5. Naturkautschuk.

6. Mischungen (Polyblends) der vorgenannten Polymeren.

7. Wässrige Emulsionen natürlicher oder synthetischer Kautschuke, wie z.B. Naturkautschuk-Latex oder Latices von carboxylierten Styrol-Butadien-Copolymeren.

Gegebenenfalls liegen diese Elastomere als Latices vor und können als solche stabilisiert werden.

Bevorzugt sind Zusammensetzungen, welche als Elastomeres ein Polydien, wie Polybutadien-Kautschuk, ein halogenhaltiges Polymer, wie Polyvinylidenfluorid, oder ein Polyurethan enthalten.

Die erfindungsgemässen Zusammensetzungen enthalten zweckmässig 0,01-10 Gew.-% an Verbindungen der Formeln I oder/und II als Stabilisatoren, bezogen auf das Elastomer bzw. auf den Schmierstoff, insbesondere 0,05-5,0 Gew.-%. Gemische dieser Verbindungen können ebenfalls eingesetzt werden.

Die Einarbeitung in die Elastomeren kann beispielsweise durch Einmischen der Substanzen der Formeln I oder/und II und gegebenenfalls weiterer Additive nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels erfolgen. Die Verbindungen der Formeln I oder II können auch in Form eines Masterbatches, der diese Verbindungen beispielsweise in einer Konzentration von 2,5 bis 25 Gew.-% enthält, den zu stabilisierenden Kunststoffen zugesetzt werden.

Die erfindungsgemässen Elastomer-haltigen Zusammensetzungen können in verschiedenster Form angewendet werden, z.B. als Folien, Fasern, Bändchen, Formmassen, Profile oder als Bindemittel für Lacke, Klebstoffe oder Kitte.

In der Praxis können die erfindungsgemässen Elastomer-haltigen Zusammensetzungen weitere Additive enthalten. Als weitere Additive sind beispielsweise zu nennen:

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tricyclohexylphenol, 2,6-Di-tert.butyl-4-methoxymethylphenol.

1.2 Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol.

1.3. Hydroxylierte Thiodiphenylether, z.B. 2,2'-Thio-bis-(6-tert.-butyl-4-methylphenol), 2,2'-Thio-bis-4-octylphenol), 4,4'-Thio-bis-(6-tert.butyl-3-methylphenol), 4,4'-Thio-bis-(6-tert.butyl-2-methylphenol).

1.4. Alkyliden-Bisphenole, z.B. 2,2'-Methylen-bis-(6-tert.butyl-4-methylphenol), 2,2'-Methylen-bis-(6-tert.butyl-4-ethylphenol), 2,2'-Methylen-bis[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis[6-(α-methylbenzyl)-4-nonylphenol], 2,2'-Methylen-bis-[6-(α,α-dimethylbenzyl)-4-nonylphenol], 4,4'-Methylen-bis-(2,6-di-tert.butylphenol), 4,4'-Methylen-bis-(6-tert.butyl-2-methylphenol), 1,1-Bis-(5-tert.-butyl-4-hydroxy-2-methylphenyl)-butan, 2,6-Di-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis[3,3-bis-(3'-tert.butyl-4'-hydroxyphenyl)-butyrat], Di-(3-tert.butyl-4-hydroxy-5-methylphenyl)-dicyclopentadien, Di[2-(3'-tert.butyl-2'-hydroxy-5'-methyl-benzyl)-6-tert.butyl-4-methyl-phenyl]-terephthalat.

1.5 Benzylverbindungen, z.B. 1,3,5-Tri-(3,5-di-tert.butyl-4-hydroxybenzyl)2,4,6-trimethylbenzol, Di-(3,5-di-tert.butyl-4-hydroxybenzyl)-sulfid, 3,5-Di-tert.butyl-4-hydroxybenzyl-mercaptoessigsäureisooctylester, Bis-(4-tert.butyl-3-hydroxy-2,6-dimethylbenzyl)-dithiol-terephthalat,1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, 3,5-Di-tert.butyl-4-hydroxybenzyl-phosphonsäure-monoethylester, Calcium-salz.

1.6. Acylaminophenole, z.B. 4-Hydroxy-laurinsäureanilid, 4-Hydroxystearinsäureanilid, 2,4-Bis-octylmercap-to-6-(3,5-di-tert.butyl-4-hydroxyanilino)-s-triazin, N-(3,5-di-tert.butyl-4-hydroxyphenyl)-carbaminsäureoctylester.

1.7. Ester der β-(3,5-Di-tert.butyl-4-hydroxyphenyl)propionsäure mit ein- oder mehrwertigen Alkoholen, wie z. B. mit Methanol, Octadecanol, 1,6-Hexandiol, Neopentylglycol, Thiodiethylenglycol, Diethylenglycol, Triethylenglycol, Pentaerythrit, Tris-hydroxyethyl-isocyanurat, Di-hydroxyethyl-oxalsäurediamid.

1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-hydroxyethyl-isocyanurat, Di-hydroxyethyl-oxalsäurediamid.

1.9. Amide der β-(3,5-Di-tert.butyl-4-hydroxyphenyl)-propionsäure, wie z.B. N,N'-Di-(3,5-di-tert.butyl-4-hydroxyphenylpropionyl)-hexamethylendiamin, N,N'-Di-(3,5-di-tert.butyl-4-hydroxyphenylpropionyl)-tri-methylendiamin, N,N'-Di-(3,5-di-tert.butyl-4-hydroxyphenylpropionyl)-hydrazin.

2. UV-Absorber und Lichtschutzmittel

2.1. 2-(2'-Hydroxyphenyl)benztriazole, wie z.B. das 5'-Methyl-, 3',5'-Di-tert.butyl-, 5'-tert.Butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.-amyl-, 3',5'-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert.Butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert. butyl-4-hydroxybenzoesäure-2,4-ditert. butylphenylester, 3,5-Di-tert.butyl-4-hydroxybenzoesäurehexadecylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestem, wie vom Methyl- oder Ethylester, Nickelkomplexe von Ketoximen, wie von 2-Hydroxy-4-methyl-phenylundecylketoxim, Nickelkomplexe des 1-Phenyl-4-lauroyl-5-hydroxypyrazols, gegebenenfalls mit zusätzlichen Liganden.

2.6. Sterisch gehinderte Amine, wie z.B. Bis-(2,2,6,6-tetramethylpiperidyl)sebacat, Bis-(1,2,2,6,6-pentamethylpiperidyl)-sebacat, n-Butyl-3,5-di-tert.butyl-4-hydroxybenzyl-malonsäure-bis(1,2,2,6,6,-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetracarbonsäure, 1,1'-(1,2-Ethandiyl)-bis-(3,3,5,5-tetramethyl-piperazinon).

2.7. Oxalsäurediamide, wie z.B. 4,4'-Di-octyloxy-oxanilid, 2,2'-Dioctyloxy-5,5'-di-tert.butyl-oxanilid,2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o- und p-Methoxy- sowie von o- und p-Ethoxy-di-substituierten Oxaniliden.

3. Metalldesaktivatoren, wie z.B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythritdiphosphit,

Tristearyl-sorbit-triphosphit, Tetrakis-(2,4-di-tert.butylphenyl)-4,4'-biphenylen-di-phosphonit, 3,9-Bis-(2,4-di-tert. butylphenoxy-2,4,8,10-tetraoxa-3,9-diphosphaspiro[5.5]undecan.

5. Peroxidzerstörende Verbindungen, wie z.B. Ester der β-Thio-dispropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, optische Aufheller, Flammschutzmittel, Antistatika, Treibmittel, Wachse, Oele oder organische Lösungsmittel.

Als Schmierstoffe können die erfindungsgemässen Zusammensetzungen Schmiermittel auf der Basis von Mineralölen oder synthetischen Oelen enthalten.

Die in Frage kommenden Schmierstoffe sind dem Fachmann geläufig und z.B. im "Schmiermittel Taschenbuch" (Hüthig Verlag, Heidelberg, 1974) beschrieben.

Erfindungsgemässe Schmierstoff-Formulierungen können zusätzlich noch andere Additive enthalten, die zugegeben werden, um gewisse Gebrauchseigenschaften zu verbessern, wie z.B. wietere Antioxidantien, Metallpassivatoren, Rostinhibitoren, Viskositäts-Index-Verbesserer, Stockpunkterniedriger. Dispergiermittel/Tenside und Verschleisschutzadditive.

Beispiele für Antioxidantien können aus der zuvor für Elastomerhaltige Zusammensetzungen genannten Aufzählung unter Punkt 1 entnommen werden.

Als weitere Additive für die Erfindungsgemässen Schmierstoff-Zusammensetzungen sind beispielsweise zu nennen:

Beispiele für aminische Antioxidantien:

N,N'-Di-isopropyl-p-phenylendiamin
N,N'-Di-sec-butyl-p-phenylendiamin
N,N'-Bis(1,4-dimethyl-pentyl)-p-phenylendiamin
N,N'-Bis(1-ethyl-3-methyl-pentyl)-p-phenylendiamin
N,N'-Bis(1-methyl-heptyl)-p-phenylendiamin
N,N'-Diphenyl-p-phenylendiamin
N,N'-Di-(naphthyl-2)-p-phenylendiamin
N-Isopropyl-N'-phenyl-p-phenylendiamin
N-(1,3-Dimethyl-butyl)-N'-phenyl-p-phenylendiamin
N-(1-Methyl-heptyl)-N'-phenyl-p-phenylendiamin
N-Cyclohexyl-N'-phenyl-p-phenylendiamin
4-(p-Toluol-sulfonamido)-diphenylamin
N,N'-Dimethyl-N,N'-di-sec-butyl-p-phenylendiamin
Diphenylamin
4-Isopropoxy-diphenylamin
N-Phenyl-1-naphthylamin
N-Phenyl-2-naphthylamin
octyliertes Diphenylamin
4-n-Butylaminophenol
4-Butyrylamino-phenol
4-Nonanoylamino-phenol
4-Dodecanoylamino-phenol
4-Octadecanoylamino-phenol
Di-(4-methoxy-phenyl)-amin
2,6-Di-tert-butyl-4-dimethylamino-methyl-phenol
2,4'-Diamino-diphenylmethan

4,4'-Diamino-diphenylmethan
N,N,N',N'-Tetramethyl-4,4'-diamino-diphenylmethan
1,2-Di-[(2-methyl-phenyl)-amino]-ethan
1,2-Di-(phenylamino)-propan
(o-Tolyl)-biguanid
Di-[4-(1',3'-dimethyl-butyl)-phenyl]amin
tert-octyliertes N-Phenyl-1-naphthylamin
Gemisch aus mono- und dialkylierten tert-Butyl-/tert-Octyldiphenylaminen.

Beispiele für Metallpassivatoren sind:

für Kupfer, z.B.:
Triazol, Benztriazol und deren Derivate, 2-Mercaptobenzthiazol, 2,5-Dimercaptothiadiazol, Salicyliden-propylendiamin, Salze von Salicylaminoguanidin.

Beispiele für Rost-Inhibitoren sind:

a) Organische Säuren, ihre Ester, Metallsalze und Anhydride, z.B.:
N-Oleoyl-sarcosin, Sorbitan-mono-oleat, Blei-naphthenat, Dodecenylbernsteinsäure-anhydrid, Alkenylbernsteinsäure-Halbester, 4-Nonylphenoxy-essigsäure.
b) Stickstoffhaltige Verbindungen, z.B.:

I. Primäre, sekundäre oder tertiäre aliphatische oder cycloaliphatische Amine und Amin-Salze von organischen und anorganischen Säuren, z.B. öllösliche Alkylammoniumcarboxylate.
II. Heterocyclische Verbindungen, z.B.: Substituierte Imidazoline und Oxazoline.

c) Phosphorhaltige Verbindungen, z.B.:
Aminsalze von Phosphorsäurepartialestern.
d) Schwefelhaltige Verbindungen, z.B.:
Barium-dinonylnaphthalin-sulfonate, Calciumpetroleum-sulfonate.

Beispiele für Viskositätsindex-Verbesserer sind z.B.:

Polymethacrylate, Vinylpyrrolidon/Methacrylat-Copolymere, Polybutene, Olefin-Copolymere, Styrol/Acrylat-Copolymere.

Beispiele für Stockpunkterniedriger sind z.B.:

Polymethacrylat, alkylierte Naphthalinderivate.

Beispiele für Dispergiermittel/Tenside sind z.B.:

Polybutenylbernsteinsäure-imide, Polybutenylphosphonsäurederivate, basische Magnesium-, Calcium-, und Bariumsulfonate und -phenolate.

Beispiele für Verschleissschutz-Additive sind z.B.:

Schwefel und/oder Phosphor und/oder Halogen enthaltende Verbindungen, wie geschwefelte pflanzliche Oele, Zinkdialkyldithiophosphate, Tritolyl-phosphat, chlorierte Paraffine, Alkyl- und Aryldisulfide.

Einige Verbindungen der Formel I sind bekannt. Die Verbindungen der Formel I, welche neu sind, sowie die Verbindungen der Formel II sind ebenfalls Gegenstand der vorliegenden Erfindung.

Die Erfindung betrifft daher auch Verbindungen der Formeln Ia oder II

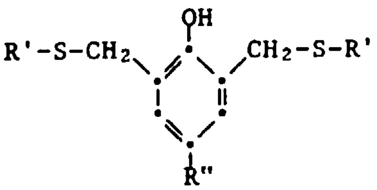 (Ia) 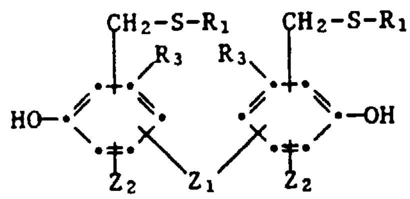 (II),

worin

die Reste R' gleich oder verschieden sind und unabhängig voneinander $C_8$-$C_{20}$-Alkyl bedeuten, oder $C_1$-$C_{20}$-Alkyl, welches durch 1 oder 2 Hydroxylgruppen substituiert oder/und durch -O- unterbrochen ist, oder $C_1$-$C_4$-Alkylen-CO-$NR''_2$ $R_4$ oder $C_5$-$C_{12}$-Cycloalkyl, bedeuten, R" Methyl, Ethyl oder ein verzweigtes $C_3$-$C_{20}$-Alkyl, Allyl, Methallyl, Propargyl oder Benzyl ist und $R_1$, $R''_2$, $R_3$, $R_4$, $Z_1$ und $Z_2$ die zuvor genannte Bedeutung haben, mit der Massgabe, dass die Phenole der Formel II in m-Stellung zur OH-Gruppe frei von der funktionellen Gruppe -$CH_2$-S-$R_1$ sind, und der Maßgabe daß, wenn R" für Methyl oder tert-Butyl steht, R' nicht Octyl ist.

Die bereits zuvor gegebenen beispielhaften und bevorzugten Bedeutungsmöglichkeiten für $R_1$ und $R_2$ in der Formel I gelten sinngemäss auch für R' und R" in der Formel Ia. Das gleiche gilt für die Symbole $R_1$, $R'_2$, $R_3$, $R_4$, $Z_1$ und $Z_2$.

Bevorzugt sind Verbindungen der Formel Ia, worin die Reste R' die gleiche Bedeutung haben und insbesondere für $C_8$-$C_{20}$-Alkyl stehen.

Ebenfalls bevorzugt sind Verbindungen der Formel Ia, worin R" Methyl, Ethyl oder ein verzweigtes $C_3$-$C_{20}$-Alkyl ist. Bevorzugt sind ferner Verbindungen der Formel II, welche der Formel IIa

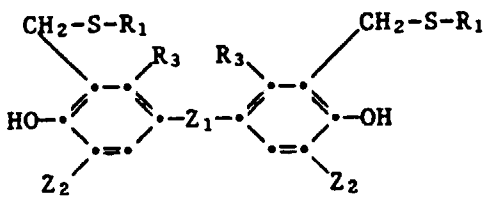 (IIa)

entsprechen, worin $R_1$, $R_3$, $Z_1$ und $Z_2$ die zuvor genannte Bedeutung haben.

Besonders bevorzugt sind Verbindungen der Formel IIa, worin $R_3$ Wasserstoff und $Z_1$ -$C(Z_3)(Z_4)$-bedeuten, wobei $Z_3$ und $Z_4$ die zuvor genannte Bedeutung haben. Ganz besonders bevorzugt sind solche Verbindungen, worin $Z_2$ den Rest -$CH_2$-S-$R_1$ bedeutet.

Die Herstellung der bekannten und neuen Verbindungen der Formeln I, Ia und II erfolgt nach an sich bekannten Methoden, wie sie beispielsweise in der EP-A-0 165 209 und in der US-A-3 227 677 beschrieben sind. Sie können aber auch durch Umsetzung eines Phenols der Formeln III oder IV

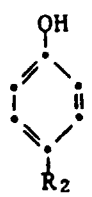 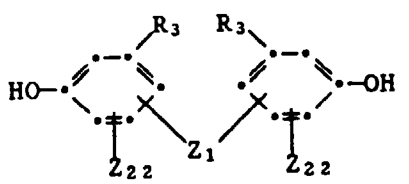 ,

(III) (IV)

worin $R_2$, $R_3$ und $Z_1$ die zuvor genannte Bedeutung haben und $Z_{22}$ Wasserstoff oder $C_1$-$C_{20}$-Alkyl ist, mit Formaldehyd oder einer unter den Reaktionsbedingungen Formaldehyd freisetzenden Verbindung und mit mindestens einem Merkaptan $R_1$-SH in Gegenwart einer Base gewonnen werden, wobei die Base Mono-, Di- oder Trimethylamin oder Mono- oder Diethylamin ist.

Bevorzugt wird Mono- oder Dimethylamin und insbesondere Dimethylamin eingesetzt. Die Base kann z.B. in Form einer 10 - 35 %-igen Lösung in Ethanol, Methanol oder anderen niederen Alkoholen oder in reiner Form verwendet werden. Dimethylamin kann auch als Gas eingesetzt werden. Die Base kann beispielsweise in einer Menge von 1-50 Mol %, vorzugsweise 2-30 Mol % und insbesondere 5-20 %, bezogen auf das Merkaptan, eingesetzt werden.

Die Umsetzung kann in Gegenwart eines Lösungsmittels durchgeführt werden.

Geeignete Lösungsmittel sind z.B. Alkohole mit ein bis sechs Kohlenstoffatomen, wie beispielsweise Methanol, Ethanol, Propanol, Butanol, Pentanol oder Hexanol. Man kann aber auch Diole, Polyole sowie deren Ether verwenden, wie beispielsweise Glykol, Glycerin, Polyethylenglycol. Die Reaktion lässt sich auch in polaren aprotischen Lösungsmitteln, wie beispielsweise Dimethylformamid oder Dimethylsulfoxid durchführen, oder es können hochsiedende aromatische oder aliphatische, gegebenenfalls chlorierte Kohlenwasserstoffe, wie beispielsweise Toluol, Ligroin oder Chlorbenzol, eingesetzt werden. Bevorzugt wird Dimethylformamid verwendet, welches gegebenenfalls mit einem der

zuvor genannten niederen Alkohole oder chlorierten Kohlenwasserstoffe verdünnt ist.

Verbindungen, die unter Reaktionsbedingungen Formaldehyd bilden, sind beispielsweise Paraformaldehyd oder Hexamethylentetramin.

Die Reaktanden Phenol, Formaldehyd und Merkaptan können in stöchiometrischen Mengen eingesetzt werden. Mitunter kann es aber von Vorteil sein, wenn der Formaldehyd und/oder das Merkaptan im Ueberschuss eingesetzt werden. Es können auch Gemische von Phenolen und/oder Merkaptanen umgesetzt werden.

Das Verfahren kann vorteilhafterweise bei Temperaturen von 80-160°C, bevorzugt von 90-150°C und insbesondere von 90-130°C und gegebenenfalls unter Druck (z.B. 0,01 bis 5 bar) durchgeführt werden. In Abwesenheit eines Lösungsmittels wird die Umsetzung bevorzugt unter Druck ausgeführt.

Die Reaktionszeiten können je nach spezifischem Phenol und Merkaptan schwanken und betragen z.B. 1 bis 24, insbesondere 1 bis 6 Stunden. Das Reaktionsgemisch wird zweckmässig in einer Stickstoffatmosphäre unter Rückfluss erhitzt.

Alle Ausgangsprodukte sind bekannte Verbindungen und können nach bekannten Verfahren hergestellt werden. Sie sind zum Teil auch im Handel erhältlich.

Die folgenden Beispiele erläutern die Erfindung weiter.

Herstellungsbeispiele

Beispiel 1: Herstellung von 2,6-Bis-(n-octylthiomethyl)-4-t-butylphenol

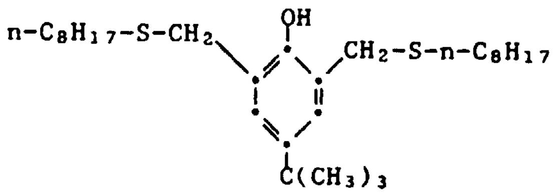

Ein Gemisch von 22,5 g 4-t-Butylphenol, 18,0 g Paraformaldehyd, 43,9 g n-Octanthiol, 4,0 g 33 %-igem ethanolischem Dimethylamin und 23 ml N,N-Dimethylformamid werden in einem Sulfierkolben mit Rückflusskühler und mechanischem Rührer unter Stickstoff während 3 Stunden unter Rückfluss erhitzt. Die Innentemperatur beträgt 110°C.

Das Rohprodukt wird in 150 ml Essigsäureethylester aufgenommen und mit 100 ml Wasser gewaschen. Nach Eindampfen der organischen Phase zur Trockene erhält man 51 g (97 % der Theorie) 2,6-Bis-(n-octylthiomethyl)-4-t-butylphenol als farbloses Oel.

| Analysewerte: | |
|---|---|
| Berechnet: | 13,74 % S |
| Gefunden: | 13,44 % S. |

Beispiel 2: Herstellung von 2,6-Bis-(n-octylthiomethyl)-4-(1',1',3',3'-tetramethylbutyl)-phenol

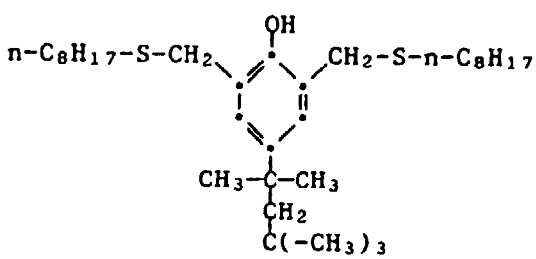

Man geht wie in Beispiel 1 vor, jedoch unter Verwendung von 0,1 Mol 4-(1',1',3',3'-Tetramethylbutyl)-phenol anstelle von 4-t-Butylphenol. Man erhält 43,6 g (83 % der Theorie) des Produktes als ein farbloses Oel.

| Analysewerte: | |
|---|---|
| Berechnet: | 12,26 % S |
| Gefunden: | 12,28 % S |

Beispiel 3: Herstellung von 2,2-Bis-[4',4"-dihydroxy-3',3",5',5"-tetrakis(n-octylthiomethyl)phenyl]-propan

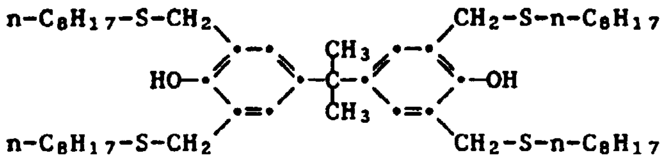

Man geht wie im Beispiel 1 vor, jedoch unter Verwendung folgenden Gemisches: 23,2 g Bisphenol A, 20,4 g Paraformaldehyd, 60,33 g n-Octanthiol, 7,5 g 33 %-iges ethanolisches Dimethylamin und 40 ml (38 g) N,N-Dimethylformamid. Die Reaktionszeit beträgt 6 Stunden. Das Rohprodukt wird in Methylenchlorid aufgenommen und mit Wasser gewaschen. Man erhält 86 g (99 % der Theorie) des Produktes als leicht gelbliches Oel.

| Analysewerte: | |
|---|---|
| Berechnet: | 14,89 % S |
| Gefunden: | 14,87 % S |

Beispiel 4: Herstellung von 2,2-Bis-[4',4"-dihydroxy-3',3",5',5"-tetrakis-(n-dodecylthiomethyl)phenyl]-propan

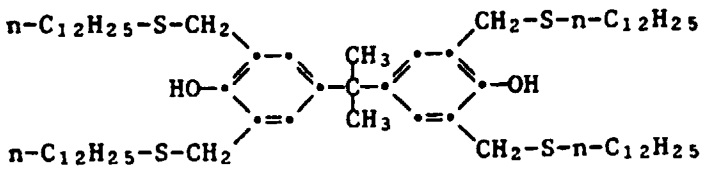

Man geht wie in Beispiel 3 vor, jedoch anstelle von n-Octanthiol wird die äquivalente Menge (in Mol) n-Dodecanthiol verwendet. Man erhält 108,9 g (99 % der Theorie) des Produktes als ein gelbliches Oel.

| Analysewerte: | |
|---|---|
| Berechnet: | 11,81 % S |
| Gefunden: | 11,67 % S. |

Beispiel 5: Herstellung eines Gemisches der Formel

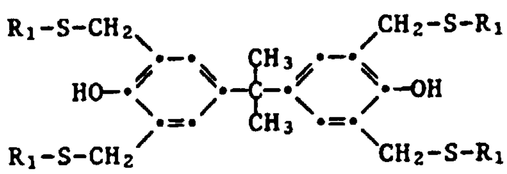

worin $R_1$ n-Octyl und/oder n-Dodecyl ist.

Man geht wie in Beispiel 3 vor, jedoch anstelle von 0,4 Mol n-Octanthiol wird ein 1:1 Gemisch von 0,2 Mol n-Octanthiol und 0,2 Mol n-Dodecanthiol eingesetzt.

Man erhält 97,9 g eines leicht gelblichen Oels. Nach chromatographischer Reinigung erhält man ein gelbliches Oel mit blumigem Geruch, welches nach der HPLC-Analyse (High Pressure Liquid Chromatography) ein statistisches Gemisch vofn 2,2-Bis-[4',4"-dihydroxy-3',3",5',5"-tetrakis($R_1$-thiomethyl)-phenyl]-propanen darstellt, worin $R_1$ n-Octyl oder n-Dodecyl ist.

| Analysewerte: (für 2x Octyl + 2x Dodecyl) | |
|---|---|
| Berechnet: | 13,17 % S |
| Gefunden: | 13,21 % S. |

<u>Beispiel 6:</u> Herstellung von Bis-[4,4'-dihydroxy-3,3',5,5'-tetrakis-(n-octylthiomethyl)phenyl]-methan

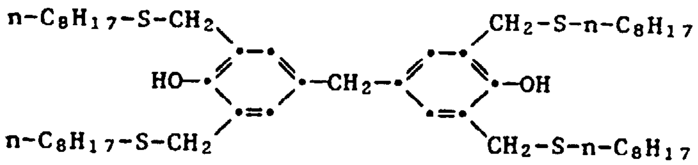

Man geht wie in Beispiel 3 vor, jedoch anstelle von Bisphenol A wird die äquivalente Menge (in Mol) Bisphenol F eingesetzt. Nach Extraktion mit Hexan erhält man 76,7 g eines orangefarbenen Oels. Gemäss $^1$H-NMR-Spektroskopie (100 MHz,$CDCl_3$) enthält es neben dem genannten Produkt noch entsprechende Reaktionsprodukte des o,p- und o,o-Isomeren von Bisphenol F als Verunreinigung.

| Analysewerte: | |
|---|---|
| Berechnet: | 15,39 % S |
| Gefunden: | 15,28 % S. |

<u>Beispiel 7:</u> Herstellung von Bis-[4,4'-dihydroxy-3,3',5,5'-tetrakis-(n-dodecylthiomethyl)-phenyl]-methan

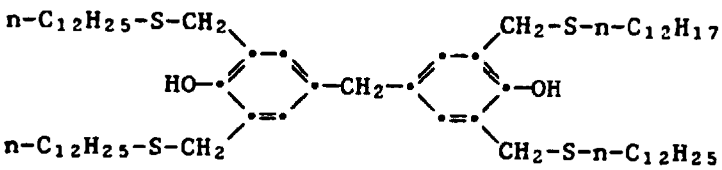

Man geht wie in Beispiel 6 vor, ausser dass anstelle von n-Octanthiol die äquivalente Menge (in Mol) n-Dodecanthiol eingesetzt wird. Man erhält ein rötliches Oel (Ausbeute: 93 %), welches in der Kälte kirstallisiert. Smp. 40 - 42°C. Auch diesmal enthält das Endprodukt neben dem genannten Hauptprodukt noch als Verunreinigung Reaktionsprodukte aus o,p- und o,o-Isomeren des Bisphenol F.

| Analysewerte: | |
|---|---|
| Berechnet: | 12,12 % S |
| Gefunden: | 12,01 % S. |

<u>Beispiel 8:</u> Herstellung von 2,2-Bis-[4',4"-dihydroxy-3',3",5',5"-tetrakis-(n-octylthiomethyl)-phenyl]-propan

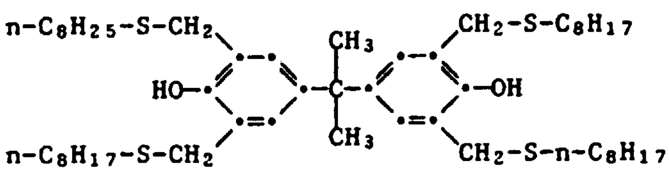

a) 23,3 g Bisphenol A, 52 g 40 %-ige wässrige Dimethylamin-lösung und 43,4 g 36 %-ige wässrige Formaldehydlösung werden während 18 Stunden auf 75°C erhitzt. Nach der üblichen Aufarbeitung durch Extraktion und Trocknung sowie Umkristallisation aus Ligroin erhält man 2,2-Bis[4',4"-dihydroxy-3',3",5',5"-tetrakis(N,N-dimethylaminomethyl)phenyl]propan als farbloses Pulver, Smp. 91 - 93°C (Ausbeute: 94 %).

b) 10,72 g des unter a) gewonnenen Produktes, 15,1 g n-Octanthiol und 0,75 g pulverisiertes Kaliumhydroxid werden auf 90°C erhitzt, bis eine klare Lösung vorliegt. Bei Raumtemperatur werden 5 ml N,N-Dimethylformamid zugegeben und anschliessend under Stickstoff während 7 Stunden auf 120°C Innentemperatur erhitzt.

Nach der üblichen Aufarbeitung erhält man 2,2-Bis-[4',4"-dihydroxy-3',3",5',5"-tetrakis-(n-octylthiomethyl)-phenyl]-propan als leicht gelbliches Oel (Ausbeute: 92,1 %).

| Analysewerte: | |
|---|---|
| Berechnet: | 14,89 % S |
| Gefunden: | 15,07 % S |

Beispiel 9: Herstellung von 2,6-Bis-(n-octylthiomethyl)-4-t-butylphenol

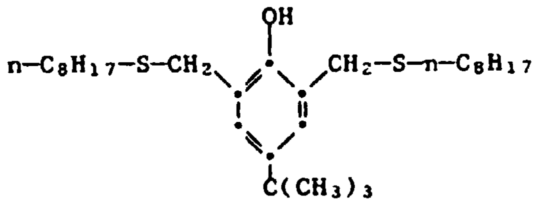

a) 177,7 g 4-t-Butylphenol, 350 ml 95 %iges Ethanol, 266,6 g 40 %-ige wässrige Dimethylaminlösung und 199,1 g 36 %-ige wässrige Formaldehydlösung werden während 12 Stunden unter Rückfluss erhitzt. Durch fraktionierte Destillation erhält man 280 g (85 % der Theorie) 2,6-Bis(N,N-dimethylaminomethyl)-4-t-butylphenol als gelbes Oel, Siedepunkt 103°C (bei 13,3 Pa).

b) 39,6 g der unter a) erhaltenen Verbindung werden mit 43,9 g n-Octanthiol während 24 Stunden bei einem leichten Vakuum von ca. 0,53 bar auf 150°C erhitzt. Man erhält nach säulenchromatographischer Reinigung 2,6-Bis-(n-octylthiomethyl)-4-t-butylphenol als gelbliches Oel (Ausbeute: 69,9 g = 98 %).

| Analysewerte: | |
|---|---|
| Berechnet: | 13,73 % S |
| Gefunden: | 13,92 % S. |

Beispiel 10: Herstellung von 2,2-Bis-[4',4"-dihydroxy-3',3",5',5"-tetrakis-(2'"-ethylhexyloxycarbonyl-methylthiomethyl) phenyl]-propan

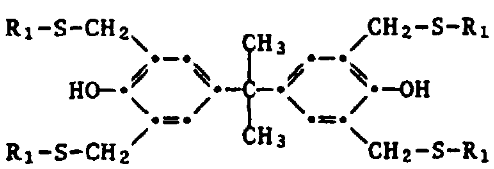

$R_1$ = $-CH_2-COO-CH_2-CH(CH_2CH_3)-(CH_2)_3-CH_3$

Man geht wie im Beispiel 3 vor, jedoch anstelle von n-Octanthiol wird die äquivalente Menge (in Mol) Thioglykolsäure-2-ethylhexylester verwendet. Man erhält das Produkt als gelbliches Oel (Ausbeute: 93,4 %).

| Analysewerte: | |
|---|---|
| Berechnet: | 11,73 % S |
| Gefunden: | 11,45 % S |

Beispiel 11: Herstellung von 2,6-Bis-(n-octylthiomethyl)-4-methylphenol

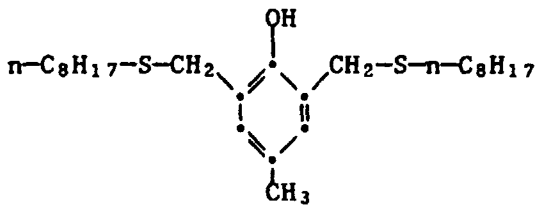

Man geht wie in Beispiel 1 vor, jedoch unter Verwendung der äquivalenten Menge (in Mol) p-Kresol anstelle von 4-t-Butylphenol. Man erhält das Produkt als gelbliches Oel (Ausbeute: 99,3 %).

| Analysewerte: | |
|---|---|
| Berechnet: | 15,10 % S |
| Gefunden: | 15,03 % S |

Beispiel 12: Herstellung von 2,6-Bis-(2'-ethylhexyloxycarbonylmethylthiomethyl)-4-t-butylphenol

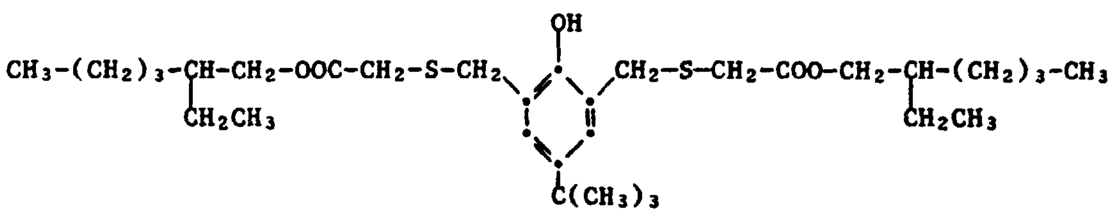

Man geht wie in Beispiel 1 vor, jedoch unter Verwendung der äquivalenten Menge (in Mol) Thioglykolsäure-2-ethylhexylester. Man erhält das Produkt als gelbliches Oel (Ausbeute: 82,5 %).

| Analysewerte: | |
|---|---|
| Berechnet: | 11,0 % S |
| Gefunden: | 10,73% S |

Die nachfolgende Tabelle 1 enthält charakteristische $^1$H-NMR spektroskopischen Daten für die Beispiele 1 bis 12 (Aryl-$\underline{CH_2}$-S-Signal). Die Angaben sind in ppm gegeben und betreffen die folgenden Strukturen:

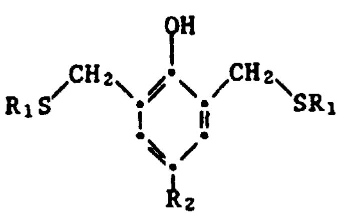

(A)

,

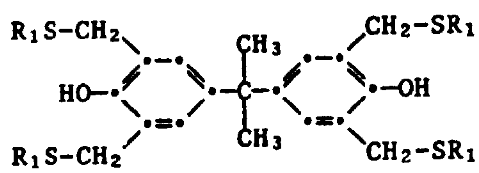

(B)

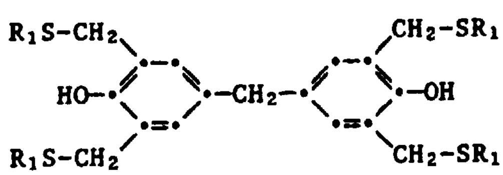

(C)

Tabelle 1

| Beispiel Nr. | Ar-$\underline{CH_2}S$** | Struktur | $R_1$ | $R_2$ |
|---|---|---|---|---|
| 1 | 3,80 (s)* | A | n-$C_8H_{17}$ | t-Butyl |
| 2 | 3,77 (s) | A | n-$C_8H_{17}$ | t-Octyl |
| 3 | 3,73 (s) | B | n-$C_8H_{17}$ | - |
| 4 | 3,73 (s) | B | n-$C_{12}H_{25}$ | - |
| 5 | 3,73 (s) | B | $C_{12}H_{25}/C_8H_{17}$ | - |
| 6 | 3,75 (s) | C | $C_8H_{17}$ | - |
| 7 | 3,75 (s) | C | $C_{12}H_{25}$ | - |
| 8 | 3,73 (s) | B | $C_8H_{17}$ | - |
| 9 | 3,80 (s) | A | n-$C_8H_{17}$ | t-Butyl |
| 10 | 3,86 (s) | B | $CH_2COO$-2-ethylhexyl | - |
| 11 | 3,78 (s) | A | n-$C_8H_{17}$ | $CH_3$ |
| 12 | 3,90 (s) | A | $CH_2COO$-2-ethylhexyl | t-Butyl |

* s = Singlett
** Ar-$\underline{CH_2}S$ = Aryl-$\underline{CH_2}$-S Signal

Anwendungsbeispiel

Stabilisierung von Polybutadien-Kautschuk (Ofenalterung)

100 g Polybutadien, welches mit 0,4 % 2,6-Di-t-butyl-p-cresol vorstabilisiert ist, werden auf einem Mischwalzwerk bei 50°C während 6 Minuten homogen mit 0,25 % 2,6-Bis-(n-octylthiomethyl)-4-t-butylphenol aus Beispiel 1 gemischt. Aus den Walzfellen werden bei 80°C Platten von 10 mm Dicke gepresst. Eine weitere Platte wird ohne Stabilisator auf dieselbe Weise hergestellt.

Die Prüfung der erfindungsgemässen Zusammensetzungen wird durch Hitzealterung in einem Umluftofen bei 80°C vorgenommen. Als Kriterium dient der während der Ofenalterung auftretende unerwünschte Gelgehalt. Dieser wird folgendermassen bestimmt:

1 Gramm Polybutadien wird über Nacht bei Raumtemperatur in 100 ml Toluol gelöst. Die Lösung wird durch eine Glasfilternutsche (Porengrösse 00) filtriert und die filtrierte Lösung zur Trockne eingedampft.

Der Gelgehalt ergibt sich aus

$$\text{Gel} = \frac{E - A}{E} \times 100\ (\%)$$

E = Einwaage (1 Gramm)
A = Gewicht des Eindampfrückstandes

Der Gelgehalt nimmt nach einer Induktionsperiode rasch zu. Als willkürliche Definition der Induktionsperiode dient die Zeit, nach welcher ein Gelgehalt von 2 % erreicht wird. Diese Induktionszeit wird durch periodische Gelgehaltsbestimmungen ermittelt.

Die Messung ergibt, dass die Probe, welche die Verbindung aus Beispiel 1 enthält, als Induktionszeit mehrere Wochen braucht gegenüber der Probe ohne Stabilisator bis das Gelgehalt von 2 % erreicht wird.

**Patentansprüche**

1. Zusammensetzungen enthaltend ein Elastomer oder einen Schmierstoff und mindestens eine Verbindung der Formeln I oder/und II

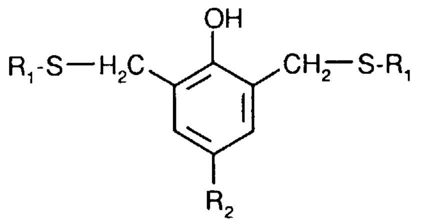 (I) 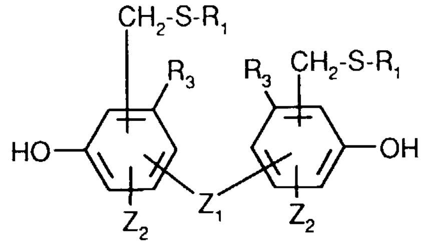 (II)

worin

die Reste $R_1$ gleich oder verschieden sind und unabhängig voneinander $C_8$-$C_{20}$-Alkyl bedeuten, welches unsubstituiert ist oder durch 1 oder 2 Hydroxylgruppen substituiert oder/und durch -O- unterbrochen ist, oder $C_1$-$C_4$-Alkylen-$COOR_{2'}$, $C_1$-$C_4$-Alkylen-CO-$NR_{2''}R_4$, $C_5$-$C_{12}$-Cycloalkyl oder Phenyl-$C_1$-$C_4$-alkyl bedeuten,
$R_2$, $R_{2'}$ und $R_{2''}$ unabhängig voneinander $C_1$-$C_{20}$-Alkyl, Allyl, Methallyl, Propargyl, $C_5$-$C_{12}$-Cycloalkyl, Phenyl oder Benzyl sind,
$R_3$ Wasserstoff, $C_1$-$C_{20}$-Alkyl, $C_2$-$C_{18}$-Alkenyl oder Halogen bedeutet,
$R_4$ Wasserstoff, $C_1$-$C_{20}$-Alkyl oder $C_2$-$C_{18}$-Alkenyl ist,
$Z_1$ für -S- oder -$C(Z_3)(Z_4)$- steht,
$Z_2$ Wasserstoff, $C_1$-$C_{20}$-Alkyl oder -$CH_2$-S-$R_1$ ist,
$Z_3$ und $Z_4$ unabhängig voneinander Wasserstoff oder Methyl bedeuten, mit der Massgabe, dass die Phenole der Formel II in m-Stellung zur OH-Gruppe frei von der funktionellen Gruppe -$CH_2$-S-$R_1$ sind.

**2.** Zusammensetzungen gemäss Anspruch 1, enthaltend ein Elastomer und mindestens eine Verbindung der Formeln I oder/und II.

**3.** Zusammensetzungen gemäss Anspruch 1, enthaltend einen Schmierstoff und mindestens eine Verbindung der Formeln I oder II, worin $R_2$ Methyl, Ethyl oder ein verzweigtes $C_3$-$C_{20}$-Alkyl, Allyl, Methallyl, Propargyl oder Benzyl ist und $R_1$, $R_3$, $Z_1$ und $Z_2$ die in Anspruch 1 genannte Bedeutung haben.

**4.** Zusammensetzungen gemäss Anspruch 1, enthaltend mindestens eine Verbindung der Formeln I oder/und II, worin die Reste $R_1$ gleich oder verschieden sind und unabhängig voneinander $C_8$-$C_{20}$-Alkyl oder $C_1$-$C_4$-Alkylen-$COOR_{2'}$ bedeuten, $R_2$ und $R_{2'}$ für $C_1$-$C_{20}$-Alkyl stehen, $Z_1$ -$CH_2$- oder -$C(CH_3)_2$- ist und $Z_2$ die in Anspruch 1 genannte Bedeutung hat.

**5.** Zusammensetzungen gemäss Anspruch 1, enthaltend mindestens eine Verbindung der Formel I, worin die Reste $R_1$ unabhängig voneinander für $C_8$-$C_{20}$-Alkylgruppen stehen, welche unsubstituiert sind, oder durch 1 oder 2 Hydroxylgruppen substituierte $C_1$-$C_{20}$-Alkylgruppen bedeuten.

**6.** Zusammensetzungen gemäss Anspruch 1, worin die Reste $R_1$ die gleiche Bedeutung haben und für $C_8$-$C_{12}$-Alkylgruppen stehen.

**7.** Zusammensetzungen gemäss Anspruch 1, enthaltend mindestens eine Verbindung der Formel IIa

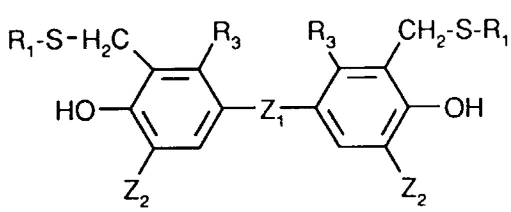 (IIa)

worin $R_1$, $R_3$, $Z_1$ and $Z_2$ die in Anspruch 1 genannte Bedeutung haben.

**8.** Zusammensetzungen gemäss Anspruch 7, worin $R_3$ Wasserstoff und $Z_1$ -$C(Z_3)(Z_4)$-bedeuten, wobei $Z_3$ und $Z_4$ die in Anspruch 1 genannte Bedeutung haben.

**9.** Zusammensetzungen gemäss Anspruch 7, worin $Z_2$ den Rest -$CH_3$-$SR_1$ bedeutet.

**10.** Zusammensetzungen gemäss Anspruch 1, worin das Elastomer ein Polydien, ein halogenhaltiges Polymer oder ein Polyurethan ist.

**11.** Zusammensetzungen gemäss Anspruch 1, enthaltend 0,01-10,0 Gew.% an Verbindungen der Formel I oder/und II, bezogen auf das Elastomer bzw. auf den Schmierstoff.

**12.** Verbindungen der Formeln Ia oder II

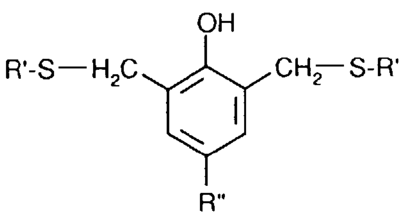 (Ia) 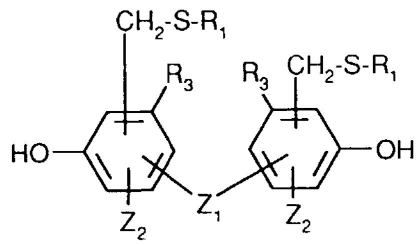 (II)

worin
die Reste R' gleich oder verschieden sind und unabhängig voneinander $C_8$-$C_{20}$-Alkyl bedeuten, oder $C_1$-$C_{20}$-Alkyl, welches durch 1 oder 2 Hydroxylgruppen substituiert oder/und durch -O- unterbrochen ist, oder $C_1$-$C_4$-Alkylen-CO-$NR_{2''}$-$R_4$ oder $C_5$-$C_{12}$-Cycloalkyl bedeuten, R" Methyl, Ethyl oder ein verzweigtes $C_3$-$C_{20}$-Alkyl, Allyl, Methallyl, Propargyl oder Benzyl ist und $R_1$, $R_{2''}$, $R_3$, $R_4$, $Z_1$ und $Z_2$ die in Anspruch 1 genannte Bedeutung haben, mit der Massgabe, dass die Phenole der Formel II in m-Stellung zur OH-Gruppe frei von der funktionellen Gruppe -$CH_2$-S-$R_1$ sind, und mit der Massgabe, dass, wenn R" für Methyl oder tert.-Butyl steht, R' nicht Octyl ist.

**13.** Verbindungen der Formel Ia gemäss Anspruch 12, worin die Reste R' die gleiche Bedeutung haben und für $C_8$-$C_{20}$-Alkyl stehen.

**14.** Verbindungen der Formel Ia gemäss Anspruch 12, worin R" Methyl, Ethyl oder ein verzweigtes $C_3$-$C_{20}$-Alkyl ist.

**15.** Verbindungen der Formel II nach Anspruch 12, worin die Verbindungen der Formel IIa

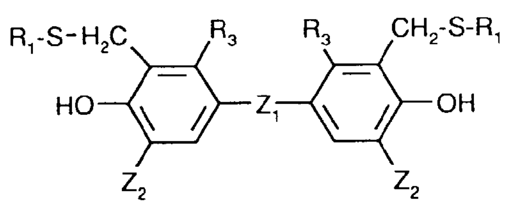 (IIa)

entsprechen, worin $R_1$, $R_3$, $Z_1$ und $Z_2$ die in Anspruch 1 genannte Bedeutung haben.

**16.** Verbindungen gemäss Anspruch 15, worin $R_3$ Wasserstoff und $Z_1$ -C($Z_3$)($Z_4$)-bedeuten, wobei $Z_3$ und $Z_4$ die in Anspruch 1 genannte Bedeutung haben.

**17.** Verbindungen gemäss Anspruch 15, worin $Z_2$ den Rest -$CH_2$-S-$R_1$ bedeutet.

**Claims**

**1.** A composition comprising an elastomer or a lubricant and at least one compound of the formulae I or/and II

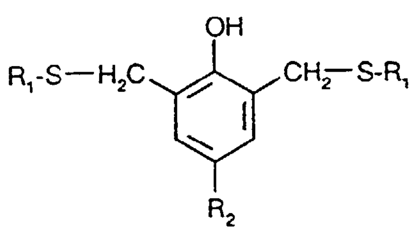 (I)

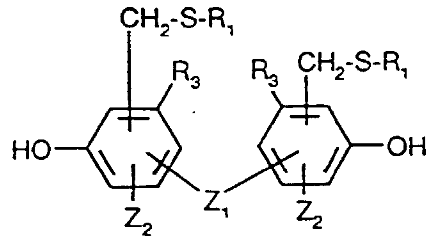

(II)

in which the radicals $R_1$ are identical or different and independently of one another are $C_8$-$C_{20}$alkyl which is unsubstituted or substituted by 1 or 2 hydroxyl groups or/and interrupted by -O-, or are $C_1$-$C_4$alkylene-$COOR_2'$, $C_1$-$C_4$alkylene-CO-$NR_2''$-$R_4$, $C_5$-$C_{12}$cycloalkyl or phenyl-$C_1$-$C_4$alkyl, $R_2$, $R_2'$ and $R_2''$ independently of one another are $C_1$-$C_{20}$alkyl, allyl, methallyl, propargyl, $C_5$-$C_{12}$cycloalkyl, phenyl or benzyl, $R_3$ is hydrogen, $C_1$-$C_{20}$alkyl, $C_2$-$C_{18}$alkenyl or halogen, $R_4$ is hydrogen, $C_1$-$C_{20}$alkyl or $C_2$-$C_{18}$alkenyl, $Z_1$ is -S- or -$C(Z_3)(Z_4)$- and $Z_2$ is hydrogen, $C_1$-$C_{20}$alkyl or -$CH_2$-S-$R_1$, $Z_3$ and Z, independently of one another being hydrogen or methyl, with the proviso that the phenols of the formula II are free of the -$CH_2$-S-$R_1$ functional group in the m-position relative to the OH group.

**2.** A composition according to claim 1, comprising an elastomer and at least one compound of the formulae I or/and II.

**3.** A composition according to claim 1, comprising a lubricant and at least one compound of the formulae I or II, in which $R_2$ is methyl, ethyl or a branched $C_3$-$C_{20}$alkyl, allyl, methallyl, propargyl or benzyl and $R_1$, $R_3$, $Z_1$ and $Z_2$ are as defined in claim 1.

**4.** A composition according to claim 1, comprising at least one compound of the formulae I or/and II, in which the radicals $R_1$ are identical or different and independently of one another are $C_8$-$C_{20}$alkyl or $C_1$-$C_4$alkylene-$COOR'_2$, $R_2$ and $R_2'$ are $C_1$-$C_{20}$alkyl, $Z_1$ is -$CH_2$- or -$C(CH_3)_2$- and $Z_2$ is as defined in claim 1.

**5.** A composition according to claim 1, comprising at least one compound of the formula I, in which the radicals $R_1$ independently of one another are $C_8$-$C_{20}$alkyl groups, which are unsubstituted, or $C_1$-$C_{20}$alkyl groups, which are substituted by 1 or 2 hydroxyl groups.

**6.** A composition according to claim 1, wherein the radicals $R_1$ are identically defined and are $C_8$-$C_{12}$alkyl groups.

**7.** A composition according to claim 1, comprising at least one compound of the formula IIa

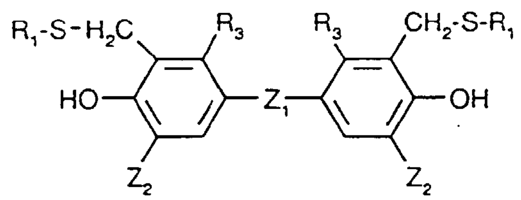

(IIa)

in which $R_1$, $R_3$, $Z_1$ and $Z_2$ are as defined in claim 1.

**8.** A composition according to claim 7, wherein $R_3$ is hydrogen and $Z_1$ is -$C(Z_3)(Z_4)$-, $Z_3$ and $Z_4$ being as defined in claim 1.

**9.** A composition according to claim 7, wherein $Z_2$ is the radical -$CH_2$-$SR_1$.

**10.** A composition according to claim 1, wherein the elastomer is a polydiene, a halogen-containing polymer or a polyurethane.

**11.** A composition according to claim 1, comprising 0.01 - 10.0% by weight of a compound of the formula I or/and II, relative to the elastomer or lubricant.

**12.** A compound of the formulae Ia or II

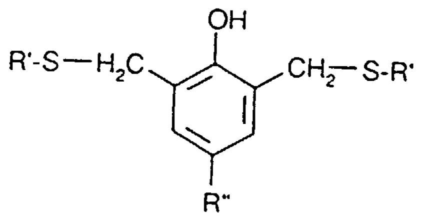 (Ia) 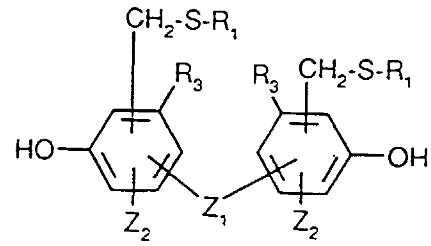 (II)

in which the radicals R' are identical or different and independently of one another are $C_8$-$C_{20}$alkyl or $C_1$-$C_{20}$alkyl which is substituted by 1 or 2 hydroxyl groups or/and interrupted by -O-, or are $C_1$-$C_4$alkylene-CO-$NR_2''R_4$ or $C_5$-$C_{12}$cycloalkyl, R" is methyl, ethyl or a branched $C_3$-$C_{20}$alkyl, allyl, methallyl, propargyl or benzyl and $R_1$, $R_2''$, $R_3$, $R_4$, $Z_1$ and $Z_2$ are as defined in claim 1, with the proviso that the phenols of the formula II are free of the -$CH_2$-S-$R_1$ functional group in the m-position relative to the OH group, and with the proviso that, when R" is methyl or tert-butyl, R' is not octyl.

**13.** A compound of the formula Ia according to claim 12, wherein the radicals R' are identically defined and are $C_8$-$C_{20}$alkyl.

**14.** A compound of the formula Ia according to claim 12, wherein R" is methyl, ethyl or a branched $C_3$-$C_{20}$alkyl.

**15.** A compound of the formula II according to claim 12, which is of the formula IIa

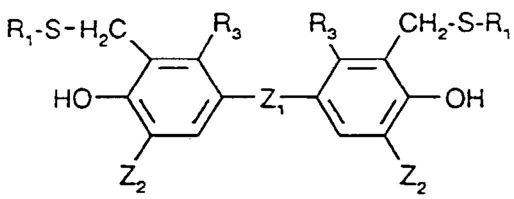 (IIa)

in which $R_1$, $R_3$, $Z_1$ and $Z_2$ are as defined in claim 1.

**16.** A compound according to claim 15, wherein $R_3$ is hydrogen and $Z_1$ is -C($Z_3$)($Z_4$)-, $Z_3$ and $Z_4$ being as defined in claim 1.

**17.** A compound according to claim 15, wherein $Z_2$ is a -$CH_2$-S-$R_1$ radical.

**Revendications**

**1.** Compositions contenant un élastomère ou un lubrifiant et au moins un composé de formules I ou/et II

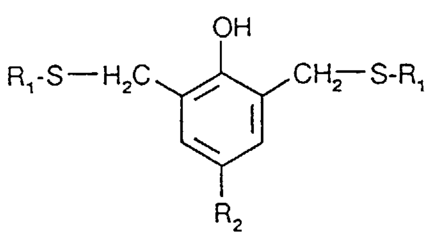 (I) 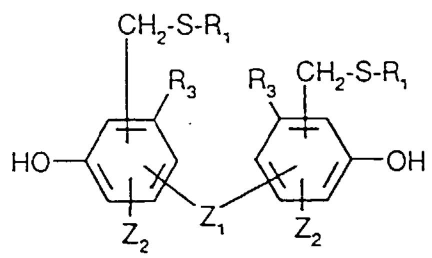 (II)

dans lesquelles

les radicaux $R_1$ sont identiques ou différents et indépendamment l'un de l'autre représentent alkyle en $C_8$-$C_{20}$, qui est non substitué ou substitué par un ou deux groupes hydroxyles ou/et interrompu par -O-, ou représente (alkylène en $C_1$-$C_4$)-$COOR_2$, (alkylène en $C_1$-$C_4$)-CO-$NR_2$-$R_4$, cycloalkyle en $C_5$-$C_{12}$ ou phénylalkyle en $C_1$-$C_4$,
$R_2$, $R_{2'}$, et $R_{2''}$, indépendamment l'un de l'autre représentent alkyle en $C_1$-$C_{20}$, allyle, méthallyle, propargyle,

cycloalkyle en $C_5$-$C_{12}$, phényle ou benzyle,
$R_3$ représente l'hydrogène, alkyle en $C_1$-$C_{20}$, alcényle en $C_2$-$C_{18}$ ou halogène,
$R_4$ représente l'hydrogène, alkyle en $C_1$-$C_{20}$ ou alcényle en $C_2$-$C_{18}$,
$Z_1$ représente -S- ou -C($Z_3$) ($Z_4$)-,
$Z_2$ représente l'hydrogène, alkyle en $C_1$-$C_{20}$ ou -$CH_2$-S-$R_1$-,
$Z_3$ et $Z_4$ indépendamment l'un de l'autre représentent l'hydrogène ou méthyle,
à la condition que les phénols de formule II soient libres du groupe fonctionnel -$CH_2$-S-$R_1$ en position m par rapport au groupe OH.

**2.** Compositions selon la revendication 1 contenant un élastomère et au moins un composé de formules I ou/et II.

**3.** Compositions selon la revendication 1, contenant un lubrifiant et au moins un composé de formules I ou II, dans lesquelles $R_2$ représente méthyle, éthyle ou un alkyle en $C_3$-$C_{20}$ ramifié, allyle, méthallyle, propargyle ou benzyle et $R_1$, $R_3$, $Z_1$ et $Z_2$ ont la signification donnée dans la revendication 1.

**4.** Compositions selon la revendication 1, contenant au moins un composé de formules I ou/et II, dans lesquelles les radicaux $R_1$ sont identiques ou différents et indépendamment l'un de l'autre représentent alkyle en $C_8$-$C_{20}$ ou (alkylène en $C_1$-$C_4$)COO$R_2$, $R_2$ et $R_{2'}$ représentent alkyle en $C_1$-$C_{20}$, $Z_1$-$CH_2$- ou -C$(CH_3)_2$- et $Z_2$ a la signification donnée dans la revendication 1.

**5.** Compositions selon la revendication 1, contenant au moins un composé de formule I, où les radicaux $R_1$ indépendamment l'un de l'autre représentent des groupes alkyle en $C_8$-$C_{20}$, qui sont non substitués ou représentent des groupes alkyle en $C_1$-$C_{20}$ substitués par un ou deux groupes hydroxyles.

**6.** Compositions selon la revendication 1, où les radicaux $R_1$ ont la même signification et représentent des groupes alkyle en $C_8$-$C_{12}$.

**7.** Compositions selon la revendication 1, contenant au moins un composé de formule IIa

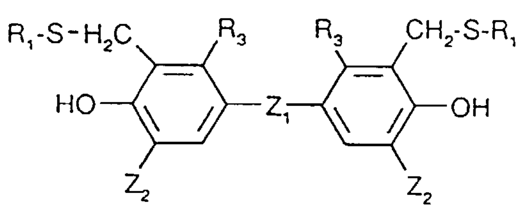

(IIa)

dans laquelle $R_1$, $R_3$, $Z_1$ et $Z_2$ ont la signification donnée dans la revendication 1.

**8.** Compositions selon la revendication 7, dans lesquelles $R_3$ représente l'hydrogène et $Z_1$-C($Z_3$) ($Z_4$)-, et $Z_3$ et $Z_4$ ayant la signification donnée dans la revendication 1.

**9.** Compositions selon la revendication 7, dans lesquelles $Z_2$ représente le radical -$CH_3$-S$R_1$.

**10.** Compositions selon la revendication 1, dans lesquelles l'élastomère est un polydiène, un polymère halogéné ou une polyuréthanne.

**11.** Compositions selon la revendication 1, contenant de 0,01 à 10,0 % en poids de composés de formules I ou/et II, par rapport à l'élastomère, respectivement au lubrifiant.

**12.** Composés de formules Ia ou II

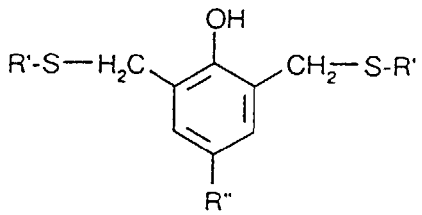

(Ia)

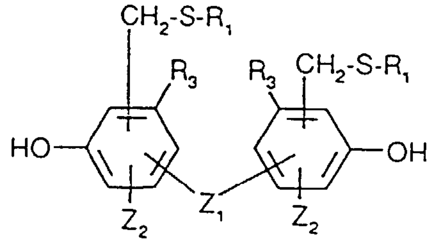

(II)

dans lesquelles les radicaux R' sont identiques ou différents, et indépendamment l'un de l'autre, représentent alkyle en $C_8$-$C_{20}$, ou alkyle en $C_1$-$C_{20}$, qui est substitué par un ou deux groupes hydroxyles ou/et ou interrompu par -O-, ou représentent (alkylène en $C_1$-$C_4$)-CO-$NR_{2''}$-$R_4$ ou cycloalkyle en $C_5$-$C_{12}$, R" représente méthyle, éthyle ou un alkyle en $C_3$-$C_{20}$ ramifié, allyle, méthallyle, propargyle ou benzyle, et $R_1$, $R_{2''}$, $R_3$, $R_4$, $Z_1$ et $Z_2$ ont la signification donnée dans la revendication 1, à la condition que les phénols de formule II soient libres du groupe fonctionnel $CH_2$-S-$R_1$ en position m par rapport au groupe OH et à la condition que si R" représente méthyle ou tert-butyle, R' ne soit pas octyle .

**13.** Composés de formule Ia selon la revendication 12, dans lesquels les radicaux R' ont la même signification et représentent alkyle en $C_8$-$C_{20}$.

**14.** Composés de formule Ia selon la revendication 12, dans lesquels R" représente méthyle, éthyle ou un alkyle en $C_3$-$C_{20}$ ramifié.

**15.** Composés de formule II selon la revendication 12, dans lesquels les composés de formule IIa

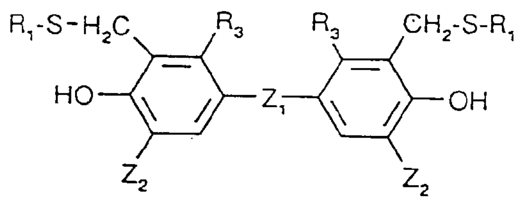

(IIa)

dans laquelle $R_1$, $R_3$, $Z_1$ et $Z_2$ ont la signification donnée dans la revendication 1.

**16.** Composés selon la revendication 15, dans lesquels $R_3$ représente l'hydrogène et $Z_1$ -C($Z_3$) ($Z_4$)-, $Z_3$ et $Z_4$ ayant les significations données dans la revendication 1.

**17.** Composés selon la revendication 15, dans lesquels $Z_2$ représente le radical -$CH_2$-S-$R_1$.